# EUROPEAN PATENT APPLICATION

(11) **EP 3 900 708 A1**
(43) Date of publication of application: **27.10.2021**
(21) Application number: 19898822.2
(22) Date of filing: 09.12.2019
(51) Int. Cl.: A61K 9/20, A61K 9/24, A61K 9/48, A61K 31/38, A61P 29/00

(54) **EXTENDED-RELEASE MEDICAL COMPOSITION CONTAINING ZALTOPROFEN**

(30) Priority: 21.12.2018 KR 20180167283
(71) Applicant: UK Chemipharm Co., Ltd., Gyeonggi-do 15598 (KR)
(72) Inventor: OH, Gi Bum, Seoul 06294 (KR); KIM, Hyun Soo, Siheung-si Gyeonggi-do 15002 (KR)
(74) Representative: Louis Pöhlau Lohrentz
(86) International application number: PCT/KR2019/017317
(87) International publication number: WO 2020/130450

(57) **Abstract**

A zaltoprofen extended-release formulation is disclosed.

## Description

### Technical Field

The present invention relates to a sustained-release pharmaceutical composition containing zaltoprofen and a method for preparing the same.

### Background Art

Zaltoprofen is a non-steroidal anti-inflammatory drug (NSAID) which is prescribed as an anti-inflammatory drug for arthritis or backache or as an anti-inflammatory drug after surgery or trauma.

Zaltoprofen has a structure of the following Formula 1:

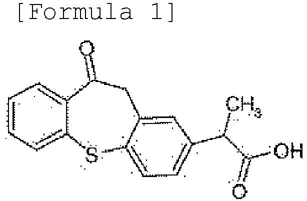

Zaltoprofen is currently manufactured and sold as an immediate-release tablet containing 80 mg of zaltoprofen, which has to be administered orally three times a day. However, a drug that has to be administered three times a day is inconvenient for patients to take.

Thus, in order to improve compliance for taking zaltoprofen and in order for zaltoprofen to exhibit prolonged efficacy, it is preferable to prepare a sustained-release formulation of zaltoprofen. However, a sustained-release formulation of zaltoprofen has not yet been developed, and hence there is an urgent need to develop a sustained-release formulation of zaltoprofen.

### [Prior Art Documents]

### [Patent Documents]

(Patent Document 1) US Patent No. 4,247,706

### DISCLOSURE

### Technical Problem

The present invention is intended to provide a sustained-release pharmaceutical composition containing zaltoprofen. In addition, the present invention is intended to provide a method for preparing a sustained-release pharmaceutical composition containing zaltoprofen.

### Technical Solution

To solve the above problem, the present invention provides a pharmaceutical composition containing: an immediate-release portion containing zaltoprofen or a pharmaceutically acceptable salt thereof and pharmaceutically acceptable additives; and a sustained-release portion containing zaltoprofen or a pharmaceutically acceptable salt thereof, a sustained-release agent and pharmaceutically acceptable additives.

The present invention also provides a method for preparing a pharmaceutical composition containing zaltoprofen, the method comprising steps of: (a) preparing an immediate-release portion containing zaltoprofen and pharmaceutically acceptable additives; (b) preparing a sustained-release portion containing zaltoprofen, a sustained-release agent and pharmaceutically acceptable additives; and (c) preparing a composition containing the immediate-release portion of step (a) and the sustained-release portion of step (b).

### Advantageous Effects

The composition containing zaltoprofen according to the present invention may release zaltoprofen in a sustained manner, and thus is a sustained-release pharmaceutical composition that may significantly improve the patient's medication compliance. In addition, since release of zaltoprofen from the immediate-release portion is not inhibited by the sustained-release portion, the drug may rapidly reaches an effective blood concentration at the initial stage after administration, so that the effect of the drug may be rapidly exhibited.

### Brief Description of Drawings

FIG. 1 graphically shows the results of a dissolution test for one example of a pharmaceutical composition according to the present invention.
FIG. 2 graphically shows the results of a dissolution test for a commercially available zaltoprofen-containing tablet as a control.

### Best Mode

The present invention is directed to a pharmaceutical composition containing: an immediate-release portion containing zaltoprofen or a pharmaceutically acceptable salt thereof and pharmaceutically acceptable additives; and a sustained-release portion containing zaltoprofen or a pharmaceutically acceptable salt thereof, a sustained-release agent and pharmaceutically acceptable additives.

In the present invention, the sustained-release portion is preferably in the form of granules containing zaltoprofen or a pharmaceutically acceptable salt thereof, a sustained-release agent and pharmaceutically acceptable additives. For example, the sustained-release portion may be granulated by a dry granulation method.

It is preferable that the granules of the sustained-release portion granulated as described above are coated with an additional sustained-release agent. For example, the granular sustained-release portion containing zaltoprofen or a pharmaceutically acceptable salt thereof, a sustained-release agent and pharmaceutically acceptable additives may be coated with a coating solution containing an additional sustained-release agent by a fluidized-bed coating method.

In the present invention, as the sustained-release agent (the sustained-release agent present in the granules or the sustained-release agent that coats the granules) that is used in the sustained-release portion art, a polymethyl methacrylate derivative or a cellulose derivative may be used.

Examples of the polymethyl methacrylate derivative include ammonio methacrylate copolymers (Eudragit RS or Eudragit RL, etc.), ethyl acrylate/methyl methacrylate copolymers (Eudragit NE, etc.), methacrylic acid copolymers (Eudragit L, Eudragit S, etc.), and amino methacrylates (Eudragit E, etc.). Examples of the cellulose derivative include hydroxypropyl methylcellulose, hydroxypropyl cellulose, and hydroxyethyl cellulose.

Preferably, an ammonio methacrylate copolymer (such as Eudragit RS or Eudragit RL) or hydroxypropyl methylcellulose is used as the sustained-release agent.

In the present invention, the immediate-release portion contains zaltoprofen or a pharmaceutically acceptable salt thereof and pharmaceutically acceptable additives, and may be used in the form of powder or in the form of granules obtained by granulating the powder. Preferably, the immediate-release portion is granulated by a dry granulation method or the like.

The immediate-release portion preferably contains at least one disintegrant selected from among crospovidone, low-substituted hydroxypropyl cellulose and croscarmellose sodium so that the immediate-release portion may disintegrate rapidly *in vivo.*

Examples of the pharmaceutically acceptable additives that are used in the present invention include diluents, binders, disintegrants, and lubricants, which are widely known in the art to which the present invention pertains.

The pharmaceutical composition of the present invention may be prepared as a single-layer tablet obtained by mixing the immediate-release portion and the sustained-release portion together; or may be prepared as a multilayer tablet or a tablet-in-tablet in which the immediate-release portion and the sustained-release portion are partitioned from each other. In addition, the pharmaceutical composition of the present invention may be prepared as a capsule containing: particles, granules, pellets or microtablets composed of the immediate-release portion; and particles, granules, pellets or microtablets composed of the sustained-release portion.

In the present invention, the content ratio between zaltoprofen present in the immediate-release portion and zaltoprofen present in the sustained-release portion may be adjusted as needed, and is preferably 1:2 to 1:4.

In addition, the present invention is directed to a method for preparing a pharmaceutical composition containing zaltoprofen, the method comprising steps of: (a) preparing an immediate-release portion containing zaltoprofen and pharmaceutically acceptable additives; (b) preparing a sustained-release portion containing zaltoprofen, a sustained-release agent and pharmaceutically acceptable additives; and (c) preparing a composition containing the immediate-release portion of step (a) and the sustained-release portion of step (b).

In the method, step (b) preferably comprises steps of: (b1) granulating zaltoprofen, the sustained-release agent and the pharmaceutically acceptable additives by a dry granulation method; and (b2) coating the granules, prepared in step (b1), with an additional sustained-release agent, for example, by a fluidized-bed coating method.

In the present invention, step (c) may comprise a step of mixing the immediate-release portion of step (a) and the sustained-release portion of step (b) and compressing the mixture into a single-layer tablet; or a step of compressing the immediate-release portion of step (a) and the sustained-release portion of step (b) into a multilayer tablet or a tablet-in-tablet. Alternatively, in the present invention, step (c) may comprise a step of preparing a capsule containing: the immediate-release portion in the form of particles, granules, pellets or microtablets; and the sustained-release portion in the form of particles, granules, pellets or microtablets.

Hereinafter, the present invention will be described in detail with reference to examples. However, the following examples serve only to illustrate the present invention, and the scope of the present invention is not limited thereto.

### Example 1: Preparation of Mixture Constituting Immediate-Release Portion

Using the components shown in Table 1 below, an immediate-release portion containing zaltoprofen was prepared.

**[Table 1]**

| Components of immediate-release portion | | |
|---|---|---|
| | Component name | Content (g) |
| Active ingredient | Zaltoprofen | 250 |
| Additive | Silicified microcrystalline cellulose | 225 |
| Additive | Polyvinylpyrrolidone | 25 |
| Additive | Magnesium stearate | 1.25 |
| Total | | 501.25 |

Zaltoprofen, silicified microcrystalline cellulose, and polyvinylpyrrolidone were mixed together in the amounts shown in Table 1 above, and then magnesium stearate was added thereto in the amount shown in Table 1 above.

The resulting mixture was granulated in a roller compactor by a dry granulation method. The granules thus prepared were sieved, and then sized and sieved again, thus preparing for granules for an immediate-release portion.

### Example 2-1: Preparation of Granules Constituting Sustained-Release Portion

Using the components shown in Table 2 below, a sustained-release portion containing zaltoprofen was prepared.

**[Table 2]**

| Components of granules for sustained-release portion | | | |
|---|---|---|---|
| | | Component name | Content (g) |
| Granules | Active ingredient | Zaltoprofen | 300 |
| | Sustained-release agent | Eudragit RS PO | 75 |
| | Additive | Calcium hydrogen phosphate dihydrate | 121.5 |
| | Additive | Magnesium stearate | 3.5 |
| Total | | | 500 |

Zaltoprofen, Eudragit RS PO, and calcium hydrogen phosphate dihydrate were mixed together in the amounts shown in Table 2 above, and then magnesium stearate was added thereto in the amount shown in Table 2 above.

The resulting mixture was granulated in a roller compactor by a dry granulation method. The granules thus prepared were sieved, and then sized and sieved again, thus preparing for granules to be used for a sustained-release portion.

### Example 2-2: Coating of Granules Constituting Sustained-Release Portion

A coating solution having the composition shown in Table 3 below was prepared, and the granules prepared in Example 2-1 were coated with the coating solution.

**[Table 3]**

| Composition of coating solution for sustained-release portion | | | |
|---|---|---|---|
| | | Component name | Content (g) |
| Coating layer | Solvent | Purified water | 117 |
| | Additive | Triethyl citrate | 5.25 |
| | Sustained-release agent | Eudragit RS 30D | 87 |
| | Additive | Silicon dioxide | 3.5 |
| | Additive | Riboflavin | 1.45 |
| Total | | | 214.2 |

Triethyl citrate, Eudragit RS 30D, silicon dioxide, and riboflavin (vitamin B2) were dissolved in purified water in the amounts shown in Table 3 above, thus preparing a coating solution. The granules prepared in Example 2-1 were spray-coated with the coating solution in a fluidized-bed granulator.

The granules coated as described above were used as granules for the sustained-release portion.

### Example 3: Preparation of Tablet

### A. Preparation of Tablet Containing 50 mg Zaltoprofen in Immediate-Release Granules and 200 mg Zaltoprofen in Sustained-Release Granules

100.2 mg of the immediate-release granules and 333.3 mg of the sustained-release granules were weighed and mixed (in this case, the amount of zaltoprofen in the immediate-release granules was about 50 mg, and the amount of zaltoprofen in the sustained-release granules was about 200 mg) .

The mixture was compressed into a single-layer tablet(A) in a tableting machine.

### B. Preparation of Tablet Containing 60 mg Zaltoprofen in Immediate-Release Granules and 200 mg Zaltoprofen in Sustained-Release Granules

120.3 mg of the immediate-release granules and 333.3 mg of the sustained-release granules were weighed and mixed (in this case, the amount of zaltoprofen in the immediate-release granules was about 60 mg, and the amount of zaltoprofen in the sustained-release granules was about 200 mg) .

The mixture was compressed into a single-layer tablet(B) in a tableting machine.

### C. Preparation of Tablet Containing 60 mg Zaltoprofen in Immediate-Release Granules and 180 mg Zaltoprofen in Sustained-Release Granules

120.3 mg of the immediate-release granules and 300 mg of the sustained-release granules were weighed and mixed (in this case, the amount of zaltoprofen in the immediate-release granules was about 60 mg, and the amount of zaltoprofen in the sustained-release granules was about 180 mg) .

The mixture was compressed into a single-layer tablet(C) in a tableting machine.

### Experimental Example 1: Dissolution Test for Tablets of Example

For tablet (A), tablet (B) and tablet (C) prepared in Example 3 above,

a dissolution test was conducted using solution II (pH 6.8 phosphate buffer/water mixture (1:1)) according to method II (paddle method) among the dissolution test methods prescribed in the Korean Pharmacopoeia (11^{th} edition).

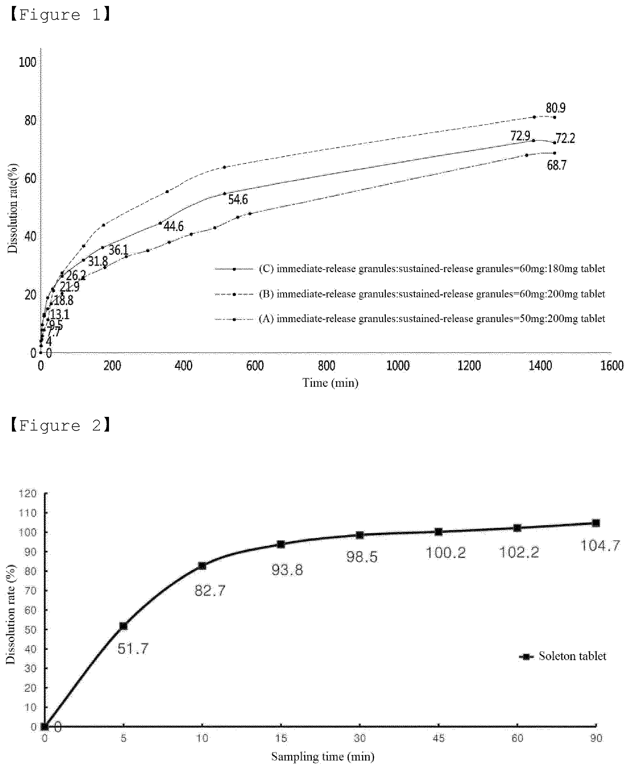

The results of the dissolution test are shown in Table 4 below and are graphically shown in FIG. 1.

**[Table 4]**

| Dissolution test results | | | | | |
|---|---|---|---|---|---|
| Tablet (A) | | Tablet (B) | | Tablet (C) | |
| Time (min) | Dissolution rate | Time (min) | Dissolution rate | Time (min) | Dissolution rate |
| 0 | 0 | 0 | 0 | 0 | 0 |
| 1 | 4 | 1 | 2.3 | 1 | 4 |
| 3 | 7.7 | 3 | 4.6 | 3 | 7.7 |
| 5 | 9.7 | 5 | 5.7 | 5 | 9.5 |
| 10 | 12.6 | 10 | 7.8 | 10 | 13.1 |
| 20 | 15.1 | 20 | 11.2 | 20 | 18.8 |
| 30 | 16.8 | 36 | 21.3 | 34 | 21.9 |
| 60 | 20.3 | 61 | 27.5 | 60 | 26.2 |
| 120 | 25.7 | 120 | 36.6 | 120 | 31.8 |
| 180 | 29.3 | 176 | 43.8 | 174 | 36.1 |
| 240 | 33 | 355 | 55.4 | 335 | 44.6 |
| 300 | 35.1 | 515 | 63.8 | 515 | 54.6 |
| 360 | 37.9 | 1383 | 81.1 | 1381 | 72.9 |
| 422 | 40.7 | 1440 | 80.9 | 1440 | 72.2 |
| 488 | 43 | | | | |
| 552 | 46.5 | | | | |
| 586 | 47.8 | | | | |
| 1362 | 67.9 | | | | |
| 1440 | 68.7 | | | | |

As a result, it can be seen that the drug was released rapidly in the initial stage by the immediate-release portion, and then released in a sustained manner over 24 hours (1,440 minutes) by the sustained-release portion.

### Experimental Example 2: Dissolution Test for Commercially Available Immediate-Release Tablet

For a currently commercially available immediate-release tablet as a control drug, a dissolution test was conducted in the same manner as in Experimental Example 1 using solution II (pH 6.8 phosphate buffer/water mixture (1:1)) according to method II (paddle method) among the dissolution test methods prescribed in the Korean Pharmacopoeia (11^{th} edition).

The results of the dissolution test are shown in Table 5 below and are graphically shown in FIG. 2.

**[Table 5]**

| Dissolution test results | | | | | | | | |
|---|---|---|---|---|---|---|---|---|
| Time | 0 | 5 | 10 | 15 | 30 | 45 | 60 | 90 |
| Average | 0 | 51.7 | 82.7 | 93.8 | 98.5 | 100.2 | 102.2 | 104.7 |

As a result of the dissolution test, it can be seen that 90% or more of the commercially available zaltoprofen tablet was dissolved within about 15 minutes in the initial stage of the test.

Taking the above results together, it can be seen that the composition of the present invention releases the drug in a sustained manner, and thus is suitable as a sustained-release formulation.

### Industrial Applicability

The composition of the present invention can be easily used as a zaltoprofen sustained-release tablet.

## Claims

1. A pharmaceutical composition containing:
an immediate-release portion containing zaltoprofen or a pharmaceutically acceptable salt thereof and pharmaceutically acceptable additives; and
a sustained-release portion containing zaltoprofen or a pharmaceutically acceptable salt thereof, a sustained-release agent and pharmaceutically acceptable additives.

2. The pharmaceutical composition of claim 1, wherein the sustained-release agent is a polymethyl methacrylate derivative or a cellulose derivative.

3. The pharmaceutical composition of claim 1, wherein the sustained-release agent is an ammonio methacrylate copolymer or hydroxypropyl methylcellulose.

4. The pharmaceutical composition of claim 1, wherein the sustained-release portion is in the form of granules containing the zaltoprofen or pharmaceutically acceptable salt thereof, the sustained-release agent and the pharmaceutically acceptable additives.

5. The pharmaceutical composition of claim 4, wherein the granules of the sustained-release portion are prepared by granulation using a dry granulation method.

6. The pharmaceutical composition of claim 4, wherein the granules of the sustained-release portion are coated with an additional sustained-release agent.

7. The pharmaceutical composition of claim 6, wherein the granules of the sustained-release portion are coated by a fluidized-bed coating method.

8. The pharmaceutical composition of claim 1, wherein the immediate-release portion contains at least one disintegrant selected from among crospovidone, low-substituted hydroxypropyl cellulose and croscarmellose sodium.

9. The pharmaceutical composition of claim 1, wherein the immediate-release portion is in the form of granules containing the zaltoprofen or pharmaceutically acceptable salt thereof and the pharmaceutically acceptable additives.

10. The pharmaceutical composition of claim 1, which is a single-layer tablet obtained by mixing the immediate-release portion and the sustained-release portion together.

11. The pharmaceutical composition of claim 1, which is a multilayer tablet or tablet-in-tablet in which the immediate-release portion and the sustained-release portion are partitioned from each other.

12. The pharmaceutical composition of claim 1, which is a capsule containing: particles, granules, pellets or microtablets composed of the immediate-release portion; and particles, granules, pellets or microtablets composed of the sustained-release portion.

13. A method for preparing a pharmaceutical composition containing zaltoprofen, the method comprising steps of:
(a) preparing an immediate-release portion containing zaltoprofen and pharmaceutically acceptable additives;
(b) preparing a sustained-release portion containing zaltoprofen, a sustained-release agent and pharmaceutically acceptable additives; and
(c) preparing a composition containing the immediate-release portion of step (a) and the sustained-release portion of step (b).

14. The method of claim 13, wherein step (b) comprises steps of:
(b1) granulating the zaltoprofen, the sustained-release agent and the pharmaceutically acceptable additives by a dry granulation method; and
(b2) coating the granules, prepared in step (b1), with an additional sustained-release agent.

15. The method of claim 14, wherein the coating in step (b) is performed by a fluidized-bed coating method.

16. The method of claim 13, wherein step (c) comprises a step of mixing the immediate-release portion of step (a) and the sustained-release portion of step (b), followed by compression into a single-layer tablet.

17. The method of claim 13, wherein step (c) comprises a step of compressing the immediate-release portion of step (a) and the sustained-release portion of step (b) into a multilayer tablet or a tablet-in-tablet.
